# EUROPEAN PATENT SPECIFICATION

(11) **EP 3 980 525 B1**
(45) Date of publication and mention of the grant of the patent: **23.07.2025**
(21) Application number: 20729107.1
(22) Date of filing: 04.06.2020
(51) Int. Cl.: C12N 5/071, A61K 35/44

(54) **COMPOSITIONS AND PROCESS FOR INTEGRATING CELLS INTO EPITHELIUM**
ZUSAMMENSETZUNGEN UND VERFAHREN ZUR INTEGRATION VON ZELLEN IN DAS EPITHEL
COMPOSITIONS ET PROCÉDÉ D'INTÉGRATION DE CELLULES DANS L'ÉPITHÉLIUM

(30) Priority: 04.06.2019 EP 19178122
(43) Date of publication of application: 13.04.2022
(73) Proprietor: Medizinische Hochschule Hannover, 30625 Hannover (DE)
(72) Inventor: MARTIN, Ulrich, 30625 Hannover (DE)
(74) Representative: Taruttis, Stefan Georg
(86) International application number: PCT/EP2020/065495
(87) International publication number: WO 2020/245277

(56) References cited:
- EP-A2- 1 214 077
- EP-B1- 1 214 077
- WO-A1-2008/143928
- WO-A1-2016/203477
- US-A1- 2005 271 697
- US-A1- 2008 153 795
- US-B2- 6 828 111
- LIU HONG ET AL: "The Effects of 17-[beta] Estradiol on Enhancing Proliferation of Human Bone Marrow Mesenchymal Stromal Cells In Vitro", STEM CELLS AND DEVELOPMENT, vol. 20, no. 5, 1 May 2011 (2011-05-01), US, pages 925 - 931, XP055640067, ISSN: 1547-3287, DOI: 10.1089/scd.2010.0125
- HILLEBRAND U ET AL: "17b-estradiol increases volume, apical surface and elasticity of human endothelium mediated by Na+/H+ exchange", CARDIOVASCULAR RESEARCH, OXFORD UNIVERSITY PRESS, GB, vol. 69, no. 4, 1 March 2006 (2006-03-01), pages 916 - 924, XP025011288, ISSN: 0008-6363, [retrieved on 20060301], DOI: 10.1016/J.CARDIORES.2005.11.025
- TANJA GROTEN ET AL: "17 [beta]-Estradiol transiently disrupts adherens junctions in endothelial cells", THE FASEB JOURNAL, vol. 19, no. 10, 1 August 2005 (2005-08-01), US, pages 1368 - 1370, XP055640093, ISSN: 0892-6638, DOI: 10.1096/fj.04-2558fje

## Description

The present invention relates to compositions for use in integrating cells into tissue, e.g. tissue of an organ, and to a process for integrating cells into extracorporeal epithelium of a tissue or organ, especially to an in vivo method of treatment of epithelium of a tissue or organ, wherein the integrating cells optionally can differ in at least one characteristic from the cells of the epithelium, e.g. the integrating cells are of heterologous origin, are heterologous or autologous cells, optionally with a genetic or epigenetic alteration introduced, or are derived from a stem cell line, e.g. pluripotent stem cells (PSCs), e.g. induced PSC (iPSCs), generated from a biopsy of the patient, e.g. autologous iPSC, or heterologous stem cells, in each case optionally with a genetic alteration introduced.

Preferably, the integrating cells are immunologically compatible to the epithelial tissue, e.g. causing no adverse immunological reaction in the recipient patient. The integrating cells can e.g. be epithelial cells including endothelial cells. From the integrating cells, cells which are derived from human embryos while destroying the embryo are excluded.

Herein, epithelium also includes endothelium, e.g. both epithelial layers and endothelial layers. The epithelium, e.g. endothelium, can be located on an internal or external surface of a tissue, which can be an organ.

Specifically, the invention relates to a combination of compositions including a suspension of cells for use in the treatment of epithelium, especially for use in the treatment of defective epithelium, especially for introducing integrating cells into the epithelium of a tissue, which may be an organ. The cells for use in the treatment of the epithelium are for integration into the epithelium. In addition to the use of the combination of compositions, which combination includes a suspension of cells, in the treatment of epithelial tissue, the invention also relates to a process for introducing integrating cells, e.g. epithelial and/or endothelial cells into epithelium, which may be endothelium, in order to produce an epithelium, which may be an endothelium, containing the integrating cells from the suspension in an integrated state. In the process, the epithelium may be part of an organ, which is preferably isolated from the body of a patient, e.g. the process preferably is an extracorporeal process. Generally, the integrating cells are used in suspension, e.g. in a single-cell or multicellular suspension. Prior to the use and prior to the process, the integrating cells are separate from the tissue containing the epithelium, e.g. separate from the patient to be treated. The suspension medium can be a cell cultivation medium, preferably a serum-free medium.

Generally, the invention also relates to a method of medical treatment of epithelium by administering to the epithelium the combination of compositions, the second composition at a time after the contact with the first composition, as described herein for the use of the combination of compounds. Generally, the combination of compositions is for use in a method of treatment described herein.

### State of the art

Groten et al., The FASEB Journal 19, No. 10, 1368 (2005) describe that 10 nM 17β-estradiol increases the permeability for FITC-labelled dextran of a cultivated monolayer of endothelial cells, namely human umbilical vein endothelial cells and uterine human microvascular endothelial cells.

WO 2016/203477 A1 describes a combination of compounds for use in conditioning a person for subsequent transplantation of progenitor cells, which combination includes a cell damaging agent and, for subsequent administration, an agent destroying resident stem cells.

EP 2 788 003 B1 describes a cell suspension from a mammalian fetal pulmonary tissue for use in treating or regenerating an epithelial, mesenchymal or endothelial tissue in a patient.

EP 3 034 087 B1 describes a cell suspension from a mammalian fetal pulmonary tissue for use in treating or regenerating an epithelial, mesenchymal or endothelial tissue in a patient who was pre-conditioned by a sublethal, lethal or supralethal conditioning protocol which includes a naphthalene treatment.

US 2005/0271697 A1 describes an implantable stent having openings that contain growth factors for improving stem cell transplantation therapy.

US 2008/0153795 A1 describes using 17β-estradiol for accelerating wound healing.

Hillebrand et al., Cardiovascular Research 916-924 (2006) describes that 17β-estradiol in a dose-dependent manner increases endothelial cell volume, apical cell surface and cell elasticity in HUVEC.

WO 2008/143928 A1 describes *inter alia* that 17β-estradiol or a selective estrogen receptor modulator or a cell can be used as a therapeutic compound for treating age-related skin conditions for inducing the epithelialization of the skin.

### Object of the invention

An object of the invention is to provide compositions for use in the treatment of epithelial tissue and a process suitable for integrating cells into epithelial tissue, e.g. for generating endothelial tissue containing the integrating cells within its endothelial tissue structure. Preferably, the compositions and the process do not have activity to destroy cells of the endothelial tissue to be treated. More preferably, the compositions and the process in addition to the cells in suspension use only natural compositions for disintegrating the existing epithelial cell layer as basis for integrating cells into an epithelial tissue.

### Description of the invention

The invention achieves the object by the features of the claims, especially by a combination of compositions comprising or consisting of a first composition containing or consisting of 17β-estradiol as the active ingredient in a buffer solution, and, as a second composition, a suspension of cells in a suspension medium, which combination of compositions is for use in the treatment of functional defects of an epithelium, e.g. of an epithelium of a tissue, which tissue may be part of or may be an organ, wherein the suspension of cells is for application at a time after the application of the 17β-estradiol to the epithelium, e.g. to the tissue containing the epithelium. The epithelium may e.g. be the epithelial layer or endothelial layer of the tissue, e.g. endothelium, e.g. of endothelialised vessels, or epithelia. The combination of compositions for use in the treatment of tissue containing epithelium, e.g. endothelium, comprises a first composition containing or consisting of 17β-estradiol as the active ingredient and a second composition comprising a suspension of cells, wherein the second composition is for contacting the epithelium of the tissue at a time after contacting the epithelium of the tissue with the first composition. The combination of compositions is for use in the treatment of epithelium, preferably for use in in vivo treatment of epithelium, the combination of composition comprising a first composition containing 17β-estradiol as the active ingredient, and a second composition comprising a suspension of cells, wherein the second composition is for contacting the epithelium at a time after the contact with the first composition. Therein generally, the time between the contacting of the epithelium with the first composition and contacting the epithelium with the second composition can e.g. be at least 1 min, e.g. at least 5 min or at least 10 min, e.g. up to 3 h, up to 2 h, or up to 60 min or up to 45 min or up to 30 min. Alternatively, the epithelium can be contacted by the second composition immediately after removal of the first composition. Generally, the epithelium is contacted with the second composition separate from the first composition, preferably the first composition is removed from the epithelium prior to contacting the epithelium with the second composition. The first composition contains or consists of 17β-estradiol as the active ingredient at a concentration suitable for adjusting the concentration of 17β-estradiol in the medium contacting the epithelium to at least 1 µM, e.g. at least 10 µM, e.g. at least 20µM or at least 30 µM 17β-estradiol, e.g. at least 50 µM, or at least 75 µM or at least 100 µM or at least 150 µM, e.g. up to 300 µM, e.g. up to 250 µM, up to 200 µM, up to 150 µM or up to 130 µM, optionally 30 to 100 µM 17β-estradiol. Optionally, in the time after contacting the epithelium with the first composition, e.g. prior to contacting the epithelium with the second composition, the endothelium may be contacted with a third composition, which is free from 17β-estradiol and does not contain added cells. Generally, the combination of compositions is for use in the treatment of the same portion of one epithelium, preferably only for treatment of the same portion of one epithelium of a patient, e.g. not for use for systemic administration or treatment. The combination of compositions preferably is for administration to a portion of a patient body only, e.g. preferably excluding systemic administration of one or all compositions of the combination of compositions. A portion of the patient body, especially the epithelium, can e.g. a portion that is perfused separately from the circulation of the patient, but preferably located in its original position within or at the patient.

The combination of compositions and the process, respectively, have the advantage of being adapted to maintain the cells of the original tissue containing the epithelium and to integrate the cells of the second composition, herein also termed integrating cells, as additional cells into the original endothelium. The additional cells contained in the resulting endothelium are provided by the second composition. The combination of compositions and the process, respectively, are suitable for use in generating a tissue having a closed epithelium, e.g. a closed epithelial cell layer comprising or consisting of the original epithelium with integrated cells of the suspension of cells. A further advantage of the combination of compositions and the process, respectively, is that it is adapted to generate a closed layer of the original epithelium having integrated cells originating from the suspension of cells within 2 to 10 h, e.g. 3 to 4 h, measured from contacting the tissue containing the epithelium with the first composition of the combination. The first composition comprising 17β-estradiol is preferably for contacting, preferably for perfusing or flushing, the tissue containing the epithelium for at least 5 min, e.g. up to 3 h, e.g. up to 90 min, up to 60 min or up to 45 min, e.g. 15 min to 3 h, e.g. 1 to 3 h, or 30 min to 2 h, preferably 20 to 30 min. Generally preferable, the first composition is for contacting the epithelium for only up to 3 h, up to 2 h, preferably for only up to 60 min, up to 45 min or up to 3 min. The second composition is preferably for contacting, preferably for perfusing, the cavities, e.g. vessels or broncheoli, in the tissue lined by the epithelium. The second composition is e.g. contacted with the endothelium for at least 15 min, e.g. up to 3 h, e.g. 30 min to 2 h, or 45 min to 90 min, e.g. 20 to 30 min. The contacting of the epithelium can also be by contacting the epithelium with an aerosol of the first and/or the second composition, e.g. by inhalation into a lung as the epithelium of the tissue, respectively as the epithelium of the organ.

The cells of the suspension of cells can be cultivated cells or, less preferred, cells obtained from disintegration of a tissue. Herein, the cells of the suspension are also referred to as integrating cells, as the combination of compositions, and respectively the process, result in the integration of the cells of the second composition into the treated tissue containing the epithelium, which herein can include endothelium, and accordingly, epithelium herein is also referred to as epithelial and/or endothelial tissue, e.g. endothelialised vessels. Herein, integrating cells that are termed epithelial cells also include endothelial cells, e.g. epithelial cells can be epithelial cells only, or endothelial cells only, or a mixture of both epithelial cells and endothelial cells, of the second composition.

The combination of the compositions comprises or consists of a first composition comprising or consisting of 17β-estradiol as the active ingredient and, for application to the epithelium separate and at a time after application of the first composition, as a second composition a suspension of cells, and optionally a solution, e.g. a medium free from 17β-estradiol and free from cells, as a third composition, which is for use for contacting the epithelium between the step contacting the epithelium with the first composition and the step of contacting the epithelium with the second composition. Accordingly, a third composition which is a medium free from 17β-estradiol and free from cells is contained in the combination and is for contacting the tissue containing the epithelium subsequent to contacting the tissue containing the endothelium with the first composition and before contacting the tissue containing the endothelium with the second composition. The third composition can e.g. be used for contacting the epithelium for at least 1 min, e.g. at least 5 or at least 10 min. The third composition can e.g. be used for contacting the epithelium for up to 60 min, e.g. up to 30 min or up to 20 min.

The first composition contains 17β-estradiol at a concentration that is sufficient for adjusting the concentration of 17β-estradiol in the medium contacting the tissue containing the epithelium, wherein the medium can be a medium suitable to maintain the viability of the tissue containing the epithelium, e.g. a physiological buffer, a cell culture medium, an organ culture medium, or blood, and to support the effect of 17β-estradiol.

The second composition comprises cells in suspension, which preferably are epithelial and/or endothelial cells or progenitor cells of epithelial and/or of endothelial cells, e.g. a stem cell line, or pluripotent stem cells (PSC), e.g. induced PSC (iPSC), e.g. generated from a biopsy of the patient, e.g. homologous iPSC, or heterologous stem cells, in each case optionally with a genetic alteration introduced. The medium of the second composition, in which the cells are in suspension, can be a medium suitable to maintain the viability of the tissue containing the epithelium to be treated, and suitable to maintain the viability of the suspended cells, e.g. a cell culture medium, an organ culture medium, or blood, or a cell-free fraction of blood. Preferably, the combination of compositions is for use in the treatment of an epithelium contained in a tissue, wherein the epithelial tissue is perfused separately from the blood circulation of the patient from which the epithelial tissue originates. For example, the epithelial tissue for perfusion is separated from the blood circulation of a patient, and is connected to an artificial medium circulation system for artificial perfusion separate from the blood circulation of a patient.

Optionally, the epithelial tissue may be located within the patient or may be located external to the patient, wherein in each case the epithelial tissue preferably is perfused separately from the blood circulation of the patient.

Accordingly, the process preferably is a process in which the tissue containing the epithelium is perfused separately from the blood circulation of the patient from whom the tissue containing the epithelium originates, e.g. the process can be an extracorporeal process, e.g. in an ex vivo perfusion system system adapted to maintain the epithelium and the tissue containing it in a living state. Ex vivo perfusion systems are e.g. known for maintaining excised organs prior to transplantation or implantation into a patient.

The combination of compositions for use in the treatment of the epithelium is preferred for tissue containing the epithelium that is perfused by a medium separate from the blood circulation from a patient from which the epithelium, respectively the tissue containing the epithelium, originates. It has been found that the active component of the first composition, 17β-estradiol, when contacting the endothelium in the medium at the concentration of at least 1 µM, preferably at least 10 µM, or at least 20 µM, more preferably at least 30 µM, e.g. up to 300 µM, e.g. up to 250 µM or up to 200 µM, e.g. 30 to 150 µM, results in the formation of gaps within the epithelium which are sufficient for the integration of suspended cells into the epithelium. It has been found that the presence of 17β-estradiol at the concentration in the medium contacting, e.g. perfusing, the tissue containing the epithelium results in a loosening or disintegration of the original structure of the epithelium, and that cells contacted with this epithelium at a time subsequent to the contact with the first composition integrate into the epithelium of the tissue. It was found that merely contacting the epithelium at a time after contact with the first composition with a suspension of the cells is sufficient for their integration, e.g. by perfusing the tissue containing the epithelium with a suspension of cells. Generally preferred, the cells in suspension are free from added 17β-estradiol, e.g. the medium of the suspension is free from 17β-estradiol.

It was found in *in vitro* tests that 3 to 5 h subsequent to contacting an epithelium with cells in suspension a closed endothelial layer was produced, containing both cells of the original epithelium and cells of the suspension of cells.

Preferably, the first composition is for contacting the epithelium, e.g. the tissue containing the epithelium, for a period of time of 5 min to 3 h, e.g. 15 min to 2 h, e.g. 10 min to 60 min, e.g. 20 to 40 min, e.g. 30 min, prior to contacting the epithelium, e.g. the tissue containing the epithelium with the cells in suspension. Optionally, the combination of compositions may comprise a third composition for application to the epithelium, e.g. to the tissue containing the epithelium, subsequent to the contact by first composition and prior to contacting the epithelium, respectively the tissue containing the epithelium, with the cells in suspension of the second composition, which third composition is free from 17β-estradiol, e.g. which third composition is a medium suitable for sustaining viability of the epithelium, e.g. a tissue culture medium, an organ culture medium, or a cell culture medium, preferably serum- free, or blood.

It was found that the combination of compositions is effective in resulting in the integration of cells of the second composition into the tissue containing the epithelium. Preferably the combination of compositions does not result in significant or sustained development of edema in the tissue containing the endothelium.

The combination of compositions for use in the treatment of tissue containing the epithelium as well as the process, respectively, according to the invention is suitable for producing a closed epithelial and/or endothelial cell layer which comprises both cells of the original epithelium and cells of the suspension of cells, wherein preferably in the closed epithelial and/or endothelial cell layer, the cells are connected by adherens junctions.

Generally, the epithelium is preferably characterized by containing adherens junctions. The epithelium, e.g. an epithelial or endothelial layer, may be part of a tissue, e.g. of an organ, which tissue can e.g. be lung tissue, e.g. a partial or complete lung, a blood vessel, an epithelial layer of the kidney or of the urinary tract.

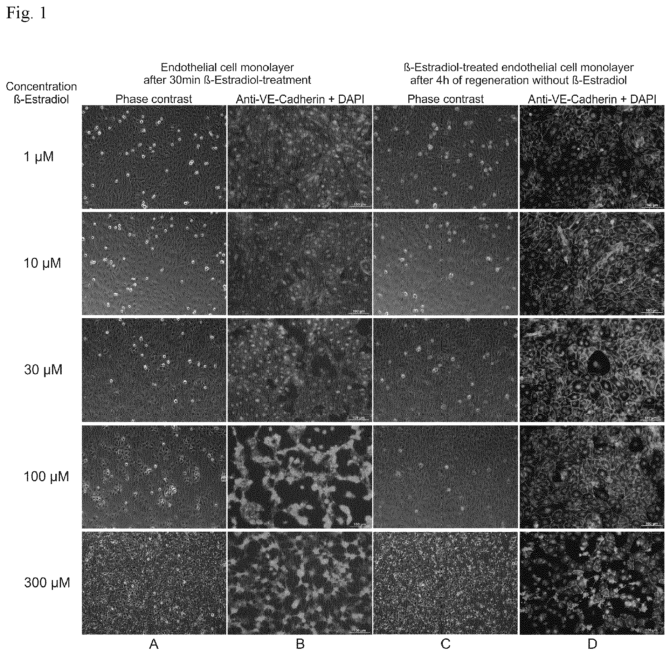

The invention is also described in greater detail with reference to the figures, which show in
- Fig. 1 microscopic pictures of monolayers of cultivated endothelial cells following contact with 17β-estradiol at different concentrations after 30 min, in column A in phase contrast and in column B after anti-VE-cadherin (green) and DAPI (blue) staining after 4 h regeneration in medium without 17β-estradiol subsequent to the incubation in the medium containing 17β-estradiol.C shows phase contrast, D shows anti-VE-cadherin and DAPI staining.

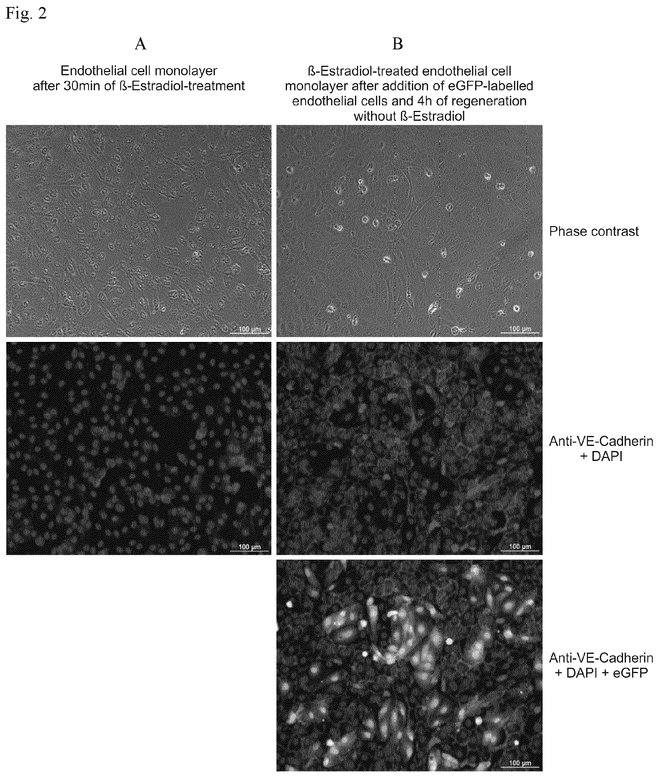

- Fig. 2 shows microscopic pictures of monolayers, in column A after 30 min in presence of 17β-estradiol, and in column B subsequent to additional contacting with cells in suspension, Upper row: phase contrast; middle row: anti-VE-cadherin staining (red) and DAPI staining (blue); lower row: anti-VE-cadherin staining (red), DAPI staining (blue) and detection newly integrated eGFP expressing endothelial cells (green).

In the figures, the scale bar is 100µm.

### Example: Integration of human primary venous endothelial cells into a venous endothelial tissue

As an example for an endothelium, human primary venous endothelial cells were cultivated in EGM-medium under cell culture conditions until reaching confluence. Then, the medium was removed from this exemplary epithelium and replaced by fresh EGM-medium containing 17β-estradiol in a concentration of 1 µM, 10 µM, 30 µM, 100 µM, or 300 µM. The concentrations of 17β-estradiol are also indicated in the rows of Fig. 1. Fig. 1 shows microscopic pictures of monolayers of the cultivated endothelial cells following the contact with 17β-estradiol at the concentrations after 30 min, in column A in phase contrast and in column B after immunological staining for anti-VE-cadherin and DAPI staining. This shows that the endothelial layer of venous endothelial cells was loosened, respectively shows disintegration after presence of at least 10 µM, preferably at least 30 µM 17β-estradiol, and at 100 µM and 300 µM 17β-estradiol for 30 min.

Subsequent to the presence of the 17β-estradiol in medium, this first composition was removed and replaced by EGM-medium without added 17β-estradiol, with subsequent incubation for 4 h under cell culture conditions. The result is depicted in Fig. 1 in column C in phase contrast, and in column D after immunological staining for VE-cadherin as an essential component of adherens junctions between cells, and DAPI staining for nuclei. The results demonstrate that that after removal of 17β-estradiol a closed layer of the endothelial cells is re-established.

A confluent layer of human primary venous endothelial cells cultivated in EGM-medium was used as a representative for endothelium. This endothelial cell layer was contacted with a first composition containing 17β-estradiol in a concentration yielding 100 µM 17β-estradiol in fresh cell culture medium. After incubating this endothelial cell layer under cell culture conditions for 30 min in the presence of 100 µM 17β-estradiol, the medium was removed and replaced by a suspension of iPSC-derived endothelial cells that were genetically manipulated to constitutively express enhanced green fluorescent green protein (eGFP) in EGM-medium without added 17β-estradiol.

The results are depicted in Fig. 2, showing in column A that contacting the endothelial cell layer with 17β-estradiol within 30 min results in loosening of the cell layer and loss of VE-cadherin expression. The result of the subsequent 4h of incubation of the endothelial cell layer with eGFP-labelled endothelial cells in suspension is depicted in Fig. 2 in the right column B. This result shows that during reestablishment of VE-cadherin expression and adherens junctions, eGFP-labelled endothelial cells of the cell suspension efficiently integrate between the original endothelial cells of the epithelium, which here is an endothelium, and form a closed endothelial cell layer comprising both endothelial cells of the original endothelium formed by the endothelial cell layer and eGFP-labelled cells of the cell suspension.

## Claims

1. Combination of compositions for use in the treatment of defective epithelium of a tissue for integrating cells into the epithelium, comprising a first composition containing 17β-estradiol as the active ingredient, and a second composition comprising a suspension of cells, wherein the second composition is for contacting the epithelium at a time after the contact with the first composition.

2. Combination of compositions for use in the treatment of defective epithelium according to claim 1, wherein the epithelium is an endothelium contained in a tissue or organ that is e.g. selected from lung, a blood vessel, kidney or urinary tract.

3. Combination of compositions for use in the treatment of defective epithelium according to one of the preceding claims, wherein the second composition is for contacting the epithelium separate from the first composition contacting the epithelium.

4. Combination of compositions for use in the treatment of defective epithelium according to one of the preceding claims, wherein the first composition and the second composition are for contacting the epithelium by perfusing, flushing, or contacting with an aerosol the tissue or organ containing the epithelium separate from the blood circulation of a patient from whom the tissue or organ containing the epithelium originates.

5. Combination of compositions for use in the treatment of defective epithelium according to one of the preceding claims, wherein the first composition contains 17β-estradiol in a concentration suitable for adjusting the concentration of 17β-estradiol in a medium contacting the epithelium to at least 1 µM 17β-estradiol.

6. Combination of compositions for use in the treatment of defective epithelium according to one of the preceding claims, wherein the first composition is for contacting the epithelium for 5 min to 3 h and the second composition is for subsequently contacting the epithelium for at least 15 min.

7. Combination of compositions for use in the treatment of defective epithelium according to one of the preceding claims, wherein the cells of the second composition are epithelial and/or endothelial cells or progenitor cells of epithelial and/or of endothelial cells, stem cells or progenitor cells, pluripotent stem cells (PSC), induced PSC (iPSC), homologous cells generated from a biopsy of the patient, and/or heterologous stem cells, in each case optionally with a genetic alteration introduced.

8. Combination of compositions for use in the treatment of defective epithelium according to one of the preceding claims, comprising a third composition which is a medium free from 17β-estradiol and free from cells, for contacting the tissue containing the epithelium subsequent to contacting the tissue containing the epithelium with the first composition and before contacting the tissue containing the epithelium with the second composition.

9. Combination of compositions for use in the treatment of defective epithelium according to one of the preceding claims, wherein the time after the contact with the first composition is at least 1 min.

10. Combination of compositions for use in the treatment of defective epithelium according to one of the preceding claims, wherein the epithelium is an endothelium.

11. Combination of compositions for use in the treatment of defective epithelium according to one of the preceding claims, wherein the epithelium is the epithelial and/or endothelial layer of a blood vessel, of a lung, of a kidney, or of the urinary tract.

12. Process for integrating cells into extracorporeal epithelium, comprising contacting the epithelium with a first composition containing 17β-estradiol as the active ingredient, and after contacting the epithelium with the first composition, contacting the epithelium with a second composition comprising a suspension of cells.

13. Process according to claim 12, wherein the extracorporeal endothelium is comprised in an extracorporeal tissue or organ, which is located outside of the body of a patient and artificially perfusing the extracorporeal tissue or organ separate from the blood circulation of the patient.

14. Process according to one of claims 12 to 13, wherein the epithelium is contacted with the first composition for at least 5 min and is subsequently contacted with the second composition for at least 15 min.

15. Process according to one of claims 12 to 14, wherein subsequent to being contacted with the first composition and prior to being contacted with the second composition, the epithelium is contacted with a third composition that is free from 17β-estradiol and free from added cells.

16. Process according to one of claims 12 to 15, wherein the extracorporeal tissue or organ is isolated from a lung or is a lung, a portion of the respiratory tract including trachea, mouth and nose, a kidney, a part of the urinary tract, a part of the small intestine, colon, stomach, esophagus, bile duct, or pancreas.

17. Method of treatment of epithelium, wherein the epithelium is located outside of the body of a patient undergoing the treatment, which epithelium is defective epithelium or defective endothelium, wherein the epithelium is perfused separately from the blood circulation of the patient undergoing the treatment , comprising contacting the epithelium for at least 5 min up to at maximum 3 h with a first composition containing 17β-estradiol as the active ingredient, and subsequently contacting the epithelium for at least 15 min with a second composition comprising a suspension of cells, wherein the second composition is for contacting the epithelium at a time after the contact with the first composition

18. Method according to claim 17 , wherein the first composition and/or the second composition is contacted with the epithelium by perfusing, flushing, or contacting with an aerosol the tisue or organ while the epithelium is separate from the blood circulation of a patient from whom the tissue or organ containing the epithelium originates.

## Patentansprüche

1. Kombination von Zusammensetzungen zur Verwendung bei der Behandlung von defektem Epithel eines Gewebes zur Integration von Zellen in das Epithel, umfassend eine erste Zusammensetzung, die 17β-Ostradiol als Wirkstoff enthält, und eine zweite Zusammensetzung, die eine Zellsuspension umfasst, wobei die zweite Zusammensetzung für den Kontakt mit dem Epithel zu einem Zeitpunkt nach dem Kontakt mit der ersten Zusammensetzung bestimmt ist.

2. Kombination von Zusammensetzungen zur Verwendung bei der Behandlung von defektem Epithel nach Anspruch 1, wobei das Epithel ein Endothel ist, das in einem Gewebe oder Organ enthalten ist, das z.B. aus Lunge, einem Blutgefäß, Niere oder Harnwegen ausgewählt ist.

3. Kombination von Zusammensetzungen zur Verwendung bei der Behandlung von defektem Epithel nach einem der vorhergehenden Ansprüche, wobei die zweite Zusammensetzung dazu bestimmt ist, das Epithel getrennt von der ersten Zusammensetzung, die das Epithel berührt, zu kontaktieren.

4. Kombination von Zusammensetzungen zur Verwendung bei der Behandlung von defektem Epithel nach einem der vorhergehenden Ansprüche, wobei die erste Zusammensetzung und die zweite Zusammensetzung dazu dienen, mit dem Epithel in Kontakt zu kommen, indem das Gewebe oder Organ, das das Epithel enthält, getrennt vom Blutkreislauf eines Patienten, von dem das Gewebe oder Organ, das das Epithel enthält, stammt, perfundiert, gespült oder mit einem Aerosol kontaktiert wird.

5. Kombination von Zusammensetzungen zur Verwendung bei der Behandlung von defektem Epithel nach einem der vorhergehenden Ansprüche, wobei die erste Zusammensetzung 17β-Ostradiol in einer Konzentration enthält, die geeignet ist, die Konzentration von 17β-Ostradiol in einem Medium, das mit dem Epithel in Kontakt steht, auf mindestens 1 µM 17β-Ostradiol einzustellen.

6. Kombination von Zusammensetzungen zur Verwendung bei der Behandlung von defektem Epithel nach einem der vorhergehenden Ansprüche, wobei die erste Zusammensetzung dazu dient, das Epithel für 5 min bis 3 h zu kontaktieren und die zweite Zusammensetzung dazu dient, das Epithel anschließend für mindestens 15 min zu kontaktieren.

7. Kombination von Zusammensetzungen zur Verwendung bei der Behandlung von defektem Epithel nach einem der vorhergehenden Ansprüche, wobei die Zellen der zweiten Zusammensetzung Epithel- und/oder Endothelzellen oder Vorläuferzellen von Epithel- und/oder Endothelzellen, Stammzellen oder Vorläuferzellen, pluripotente Stammzellen (PSC), induzierte PSC (iPSC), homologe Zellen, die aus einer Biopsie des Patienten erzeugt wurden, und/oder heterologe Stammzellen sind, jeweils gegebenenfalls mit einer eingeführten genetischen Veränderung.

8. Kombination von Zusammensetzungen zur Verwendung bei der Behandlung von defektem Epithel nach einem der vorhergehenden Ansprüche, umfassend eine dritte Zusammensetzung, die ein Medium ist, das frei von 17β-Östradiol und frei von Zellen ist, zum Inkontaktbringen des das Epithel enthaltenden Gewebes nach dem Inkontaktbringen des das Epithel enthaltenden Gewebes mit der ersten Zusammensetzung und vor dem Inkontaktbringen des das Epithel enthaltenden Gewebes mit der zweiten Zusammensetzung.

9. Kombination von Zusammensetzungen zur Verwendung bei der Behandlung von defektem Epithel nach einem der vorhergehenden Ansprüche, wobei die Zeit nach dem Kontakt mit der ersten Zusammensetzung mindestens 1 min. beträgt.

10. Kombination von Zusammensetzungen zur Verwendung bei der Behandlung von defektem Epithel nach einem der vorhergehenden Ansprüche, wobei das Epithel ein Endothel ist.

11. Kombination von Zusammensetzungen zur Verwendung bei der Behandlung von defektem Epithel nach einem der vorhergehenden Ansprüche, wobei das Epithel die Epithel- und/oder Endothelschicht eines Blutgefäßes, einer Lunge, einer Niere oder der Harnwege ist.

12. Verfahren zur Integration von Zellen in extrakorporales Epithel, umfassend das Inkontaktbringen des Epithels mit einer ersten Zusammensetzung, die 17β-Ostradiol als Wirkstoff enthält, und nach dem Inkontaktbringen des Epithels mit der ersten Zusammensetzung das Inkontaktbringen des Epithels mit einer zweiten Zusammensetzung, die eine Zellsuspension enthält.

13. Verfahren nach Anspruch 12, bei dem das extrakorporale Endothel in einem extrakorporalen Gewebe oder Organ enthalten ist, das sich außerhalb des Körpers eines Patienten befindet und das extrakorporale Gewebe oder Organ getrennt vom Blutkreislauf des Patienten künstlich perfundiert wird.

14. Verfahren nach einem der Ansprüche 12 bis 13, bei dem das Epithel mindestens 5 Minuten lang mit der ersten Zusammensetzung in Kontakt gebracht wird und anschließend mindestens 15 Minuten lang mit der zweiten Zusammensetzung in Kontakt gebracht wird.

15. Verfahren nach einem der Ansprüche 12 bis 14, bei dem Epithel nach dem Inkontaktbringen mit der ersten Zusammensetzung und vor dem Inkontaktbringen mit der zweiten Zusammensetzung mit einer dritten Zusammensetzung in Kontakt gebracht wird, die frei von 17β-Östradiol und frei von zugesetzten Zellen ist.

16. Verfahren nach einem der Ansprüche 12 bis 15, bei dem das extrakorporale Gewebe oder Organ aus einer Lunge isoliert wird oder eine Lunge, ein Teil des Respirationstrakts einschließlich Trachea, Mund und Nase, eine Niere, ein Teil des Harntrakts, ein Teil des Dünndarms, Dickdarms, Magens, der Speiseröhre, des Gallengangs oder der Bauchspeicheldrüse ist.

17. Verfahren zur Behandlung von Epithelium, bei dem sich das Epithelaußerhalb des Körpers eines Patienten befindet, der sich der Behandlung unterzieht, wobei das Epithel defektes Epithel oder defektes Endothel ist, wobei das Epithel getrennt vom Blutkreislauf des Patienten, der sich der Behandlung unterzieht, perfundiert wird, umfassend das In-Kontakt-Bringen des Epithels für mindestens 5 min bis maximal 3 h mit einer ersten Zusammensetzung, die 17β-Ostradiol als Wirkstoff enthält, und anschließend das In-Kontakt-Bringen des Epithels für mindestens 15 min mit einer zweiten Zusammensetzung, die eine Zellsuspension umfasst, wobei die zweite Zusammensetzung zum In-Kontakt-Bringen des Epithels zu einem Zeitpunkt nach dem Kontakt mit der ersten Zusammensetzung dient.

18. Verfahren nach Anspruch 17 , bei dem die erste Zusammensetzung und/oder die zweite Zusammensetzung mit dem Epithel in Kontakt gebracht wird, indem das Gewebe oder Organ perfundiert, gespült oder mit einem Aerosol in Kontakt gebracht wird, während das Epithel vom Blutkreislauf eines Patienten getrennt ist, von dem das Gewebe oder Organ, das das Epithel enthält, stammt.

## Revendications

1. Combinaison de compositions à utiliser dans le traitement de l'épithélium défectueux d'un tissu pour intégrer des cellules dans l'épithélium, comprenant une première composition contenant du 17β-estradiol comme ingrédient actif, et une seconde composition comprenant une suspension de cellules, dans laquelle la seconde composition est destinée à entrer en contact avec l'épithélium à un moment postérieur au contact avec la première composition.

2. Combinaison de compositions à utiliser dans le traitement de l'épithélium défectueux selon la revendication 1, dans laquelle l'épithélium est un endothélium contenu dans un tissu ou un organe choisi par exemple parmi les poumons, les vaisseaux sanguins, les reins ou les voies urinaires.

3. Combinaison de compositions à utiliser dans le traitement de l'épithélium défectueux selon l'une des revendications précédentes, dans laquelle la seconde composition est destinée à entrer en contact avec l'épithélium séparément de la première composition entrant en contact avec l'épithélium.

4. Combinaison de compositions à utiliser dans le traitement de l'épithélium défectueux selon l'une des revendications précédentes, dans laquelle la première composition et la seconde composition sont destinées à entrer en contact avec l'épithélium par perfusion, rinçage ou contact avec un aérosol du tissu ou de l'organe contenant l'épithélium séparé de la circulation sanguine d'un patient dont provient le tissu ou l'organe contenant l'épithélium.

5. Combinaison de compositions p à utiliser dans le traitement de l'épithélium défectueux selon l'une des revendications précédentes, dans laquelle la première composition contient du 17β-estradiol à une concentration appropriée pour ajuster la concentration de 17β-estradiol dans un milieu en contact avec l'épithélium à au moins 1 µM de 17β-estradiol.

6. Combinaison de compositions pour 'le traitement d'un épithélium défectueux selon l'une des revendications précédentes, dans laquelle la première composition est destinée à entrer en contact avec l'épithélium pendant 5 min à 3 h et la seconde composition est destinée à entrer ensuite en contact avec l'épithélium pendant au moins 15 min.

7. Combinaison de compositions à utiliser dans le traitement de l'épithélium défectueux selon l'une des revendications précédentes, dans laquelle les cellules de la deuxième composition sont des cellules épithéliales et/ou endothéliales ou des cellules progénitrices de cellules épithéliales et/ou endothéliales, des cellules souches ou des cellules progénitrices, des cellules souches pluripotentes (PSC), des PSC induites (iPSC), des cellules homologues générées à partir d'une biopsie du patient, et/ou des cellules souches hétérologues, dans chaque cas avec, éventuellement, l'introduction d'une altération génétique.

8. Combinaison de compositions à utiliser dans le traitement de l'épithélium défectueux selon l'une des revendications précédentes, comprenant une troisième composition qui est un milieu exempt de 17β-estradiol et exempt de cellules, pour la mise en contact du tissu contenant l'épithélium après la mise en contact du tissu contenant l'épithélium avec la première composition et avant la mise en contact du tissu contenant l'épithélium avec la deuxième composition.

9. Combinaison de compositions à utiliser dans le traitement de l'épithélium défectueux selon l'une des revendications précédentes, dans laquelle le temps après le contact avec la première composition est d'au moins 1 min.

10. Combinaison de compositions à utiliser dans le traitement de l'épithélium défectueux selon l'une des revendications précédentes, dans laquelle l'épithélium est un endothélium.

11. Combinaison de compositions à utiliser dans le traitement de l'épithélium défectueux selon l'une des revendications précédentes, dans laquelle l'épithélium est la couche épithéliale et/ou endothéliale d'un vaisseau sanguin, d'un poumon, d'un rein ou des voies urinaires.

12. Procédé d'intégration de cellules dans un épithélium extracorporel, comprenant la mise en contact de l'épithélium avec une première composition contenant du 17β-estradiol comme ingrédient actif, et après la mise en contact de l'épithélium avec la première composition, la mise en contact de l'épithélium avec une deuxième composition comprenant une suspension de cellules.

13. Procédé selon la revendication 12, dans lequel l'endothélium extracorporel est compris dans un tissu ou un organe extracorporel, qui est situé à l'extérieur du corps d'un patient et qui perfuse artificiellement le tissu ou l'organe extracorporel séparément de la circulation sanguine du patient.

14. Procédé selon l'une des revendications 12 à 13, dans lequel l'épithélium est mis en contact avec la première composition pendant au moins 5 min et est ensuite mis en contact avec la seconde composition pendant au moins 15 min.

15. Procédé selon l'une des revendications 12 à 14, dans lequel, après avoir été mis en contact avec la première composition et avant d'être mis en contact avec la deuxième composition, l'épithélium est mis en contact avec une troisième composition exempte de 17β-estradiol et de cellules ajoutées.

16. Procédé selon l'une des revendications 12 à 15, dans lequel le tissu ou l'organe extracorporel est isolé d'un poumon ou est un poumon, une partie de l'appareil respiratoire comprenant la trachée, la bouche et le nez, un rein, une partie de l'appareil urinaire, une partie de l'intestin grêle, du côlon, de l'estomac, de l'œsophage, des voies biliaires ou du pancréas.

17. Procédé de traitement de l'épithélium, dans laquelle l'épithélium est situé à l'extérieur du corps d'un patient subissant le traitement, lequel épithélium est un épithélium défectueux ou un endothélium défectueux, dans laquelle l'épithélium est perfusé séparément de la circulation sanguine du patient subissant le traitement, comprenant la mise en contact de l'épithélium pendant au moins 5 minutes et au maximum 3 heures avec une première composition contenant du 17β-estradiol en tant qu'ingrédient actif, puis la mise en contact de l'épithélium pendant au moins 15 min. avec une deuxième composition comprenant une suspension de cellules, la deuxième composition étant destinée à la mise en contact de l'épithélium à un moment donné après la mise en contact avec la première composition.

18. Procédé selonla revendication 17 , dans laquelle la première composition et/ou la seconde composition est mise en contact avec l'épithélium par perfusion, rinçage ou contact avec un aérosol du tissu ou de l'organe alors que l'épithélium est séparé de la circulation sanguine d'un patient dont provient le tissu ou l'organe contenant l'épithélium.
